# EUROPEAN PATENT APPLICATION

(11) **EP 2 228 016 A1**
(43) Date of publication of application: **15.09.2010**
(21) Application number: 10250430.5
(22) Date of filing: 09.03.2010
(51) Int. Cl.: A61B 17/06, A61B 17/34, A61B 17/04

(54) **Access port including multi-layer seal and suture parks**

(30) Priority: 10.03.2009 US 158824 P; 01.02.2010 US 697566
(71) Applicant: Tyco Healthcare Group LP, North Haven, CT 06473 (US)
(72) Inventor: Carter, Sally, Wallingford, CT 06492 (US)
(74) Representative: Soames, Candida Jane

(57) **Abstract**

A seal assembly configured for use with a cannula assembly includes a housing and a suture retaining member disposed in mechanical cooperation with the housing. The suture retaining member is configured to hold at least one suture and configured to help prevent the tangling of sutures. A slit seal member is disposed in mechanical cooperation with the housing. The slit seal member is configured to create a substantially fluid-tight seal in the absence of a surgical instrument inserted therethrough. An instrument seal member is disposed in mechanical cooperation with the housing. The instrument seal member is configured to create a substantially fluid-tight seal around a surgical instrument inserted therethrough.

## Description

### CROSS REFERENCE TO RELATED APPLICATION

The present application claims the benefit of and priority to U.S. Provisional Application Serial No. 61/158,824 filed on March 10, 2009, the entire contents of which are incorporated herein by reference.

### BACKGROUND

### Technical Field

The present disclosure relates to access ports for providing access into a patient's body. In particular, the present disclosure relates to access ports which are adapted to allow the introduction of surgical instrumentation into a patient's body. The access ports also include suture retaining members configured to hold sutures.

### Description of the Related Art

In laparoscopic procedures surgery is performed in the interior of the abdomen through a small incision; in endoscopic procedures surgery is performed in any hollow viscus of the body through a narrow tube or cannula inserted through a small entrance incision in the skin. Laparoscopic and endoscopic procedures generally require that any instrumentation inserted into the body be sealed, i.e. provisions must be made to ensure that gases do not enter or exit the body through the incision as, for example, in surgical procedures in which the surgical region is insufflated. Moreover, laparoscopic and endoscopic procedures often require the surgeon to act on organs, tissue, and vessels far removed from the incision, thereby requiring that any instruments used in such procedures be relatively long and narrow.

For such procedures, the introduction of a tube into certain anatomical cavities such as the abdominal cavity is usually accomplished by use of a trocar assembly made up of a cannula assembly and an obturator assembly. Since the cannula assembly provides a direct passage for surgical instrumentation from outside the patient's body to access internal organs and tissue, it is important that the cannula assembly maintain a relatively gas-tight interface between the abdominal cavity and the outside atmosphere. The cannula assembly generally includes a cannula attached to a cannula housing containing a seal assembly adapted to maintain a seal across the opening of the cannula housing.

Since surgical procedures in the abdominal cavity of the body require insufflating gases to raise the cavity wall away from vital organs, the procedure is usually initiated by use of a Verres needle through which a gas such as CO₂ is introduced into the body cavity, thereby creating a pneumoperitoneum. The gas provides a positive pressure which raises the inner body wall away from internal organs, thereby providing the surgeon with a region within which to operate and avoiding unnecessary contact with the organs by the instruments inserted through the cannula assembly. An obturator of the obturator assembly is inserted into the cannula assembly and used to puncture the abdominal wall. Following removal of the obturator assembly from the cannula assembly, laparoscopic or endoscopic surgical instruments may be inserted through the cannula assembly to perform surgery within the abdominal cavity.

Generally in the context of insufflatory surgical procedures, there are two sealing requirements for cannula assemblies. The first requirement is to provide a substantially fluid-tight seal when an instrument is not being introduced into or is not already present in the cannula. The second requirement is to provide a substantially fluid-tight seal when an instrument is being introduced into or is already present in the cannula.

In certain procedures, such as arthroscopic procedures, it is sometimes necessary to secure soft tissue to a selected bone surface either directly or indirectly via an implant typically called an anchor. These anchors are generally tied to sutures and the sutures are then tied to the soft tissue to hold it in place. Anchors may be used to attach soft tissue such as ligaments, tendons, muscles, etc., to a bone surface from which the soft tissue has become detached. Anchors may also be employed to secure soft tissue to supplementary attachment sites for reinforcement. During these kinds of procedures, surgeons may sometimes introduce multiple sutures through the cannula assembly of the trocar assembly. As a result, these sutures may tangle during the surgical procedure. In addition, identification of the suture may also be an issue.

In addition, in conjunction with an arthroscopic procedure, fluids such as saline, may be deposited into the surgical site to distend, e.g., the joint, and/or clean away debris thus presenting sealing concerns within the interior of the portal or cannula.

### SUMMARY

In accordance with the present disclosure, a seal assembly configured for use with a cannula assembly is provided. The seal assembly includes a housing, a suture retaining member, a slit seal member and an instrument seal member. The suture retaining member is disposed in mechanical cooperation with the housing and is configured to hold at least one suture to minimize the potential of suture entanglement. The suture retaining member may be disposed in mechanical cooperation with a proximal portion of the housing. The suture retaining member may have a plurality of suture parks disposed therethrough. Each suture park may be disposed adjacent to a slit defined through the suture retaining member.

The slit seal member is disposed in mechanical cooperation with the housing and is configured to create a substantially fluid-tight seal in the absence of a surgical instrument inserted therethrough. The slit seal member may be disposed in mechanical cooperation with the housing adjacent and distal to the suture retaining member. The seal assembly may also include two slit seal members wherein each slit seal member is disposed in mechanical cooperation with the housing.

The instrument seal member is disposed in mechanical cooperation with the housing and is configured to create a substantially fluid-tight seal around a surgical instrument inserted therethrough.

In accordance with the present disclosure, an access port is also provided, the access port including a housing and a seal assembly disposed within the housing. The seal assembly includes a first portion having a suture retaining member, a second portion having a slit seal member and a third portion having an instrument seal member. The housing may be configured to mechanically engage a cannula assembly.

In disclosed embodiments, the suture retaining member has a plurality of suture parks configured to hold sutures and is configured to help prevent the tangling of sutures. Each suture park may be disposed adjacent to a slit defined through the suture retaining member.

### BRIEF DESCRIPTION OF THE DRAWINGS

Various embodiments of the present disclosure are described herein with reference to the drawings wherein:
Fig. 1 is a perspective view of an access port including a suture retaining member, a slit seal member and an instrument seal member.
Fig. 2A is a top plan view of the suture retaining member of Fig. 1.
Fig. 2B is a top plan view of the slit seal member of Fig. 1.
Fig. 2C is a top plan view of the instrument seal member of Fig. 1.
Fig. 3 is a longitudinal cross-sectional view of the access port of Figs. 1 and 2, attached to a cannula assembly and having an instrument disposed therethrough.
Fig. 4 is an exploded view of the suture retaining member, the slit seal member, and the instrument seal member of the access port of Figs. 1-3.

### DETAILED DESCRIPTION

The access ports of the present disclosure, either alone or in combination with a cannula assembly, provide a substantially fluid-tight seal between a body cavity of a patient and the outside atmosphere. The access ports, or seal assemblies, of the present disclosure are configured to receive surgical instruments of varying diameter and are also configured to retain sutures therein and to help prevent the tangling of sutures. Various procedures contemplated include laparoscopic and arthroscopic procedures.

The access ports of the present disclosure contemplate the introduction of various types of instrumentation adapted for insertion through a trocar and/or cannula assembly while maintaining a substantially fluid-tight interface about the instrument to help preserve the atmospheric integrity of a surgical procedure from gas and/or fluid leakage. Examples of instrumentation include, but are not limited to, clip appliers, graspers, dissectors, retractors, staplers, laser probes, photographic devices, endoscopes and laparoscopes, tubes, and the like. Such instruments will collectively be referred to as "instruments" or "instrumentation."

In addition to instrumentation, the access ports also allow the passage of one or more sutures therethrough. When several sutures are introduced into the subject's body through a typical access port, the sutures may tangle with each other. In addition, identification of the sutures also becomes an issue. To minimize the possibility of sutures tangling with one another and to assist the surgeon in identifying particular sutures and their respective functions, the access ports incorporate suture retaining members for holding the sutures in place.

In the following description, as is traditional, the term "proximal" refers to the portion of the device closer to the operator while the term "distal" refers to the portion of the device farther from the operator.

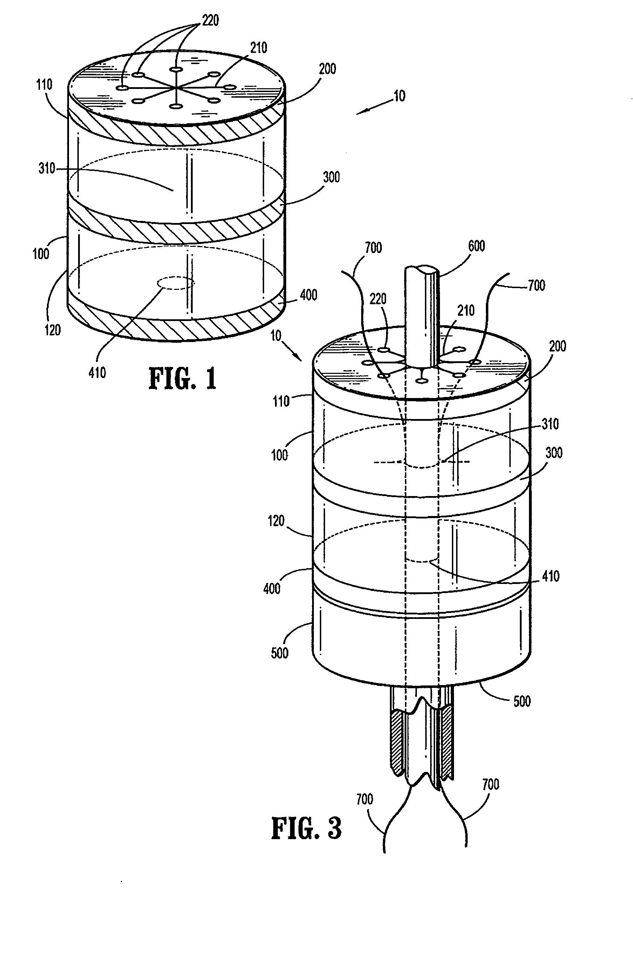

Referring now to the drawings, **Fig. 1** illustrates a seal assembly **10** including a housing **100.** Housing **100** of seal assembly **10** includes a suture retaining member **200** disposed in mechanical cooperation with housing **100,** a slit seal member **300** disposed in mechanical cooperation with housing **100,** and an instrument seal member **400** disposed in mechanical cooperation with housing **100.** It is envisioned that at least one of suture retaining member **200,** slit seal member **300** and instrument seal member **400** is fixedly attached to (e.g. monolithically formed with) housing **100.**

In the embodiment shown in **Fig. 1****,** suture retaining member **200** is disposed in mechanical cooperation with the proximal portion **110** of housing **100.** The slit seal member **300** is disposed in mechanical cooperation with housing **100** adjacent and distal to the suture retaining member **200.** The instrument seal member **400** is disposed in mechanical cooperation with the distal portion **120** of housing **100** adjacent and distal to slit seal member **300.**

It is envisioned that a second slit seal member (not shown) may also be included within housing **100** such that the slit seal members are adjacent each other. The second slit seal member would function in substantially the same manner as the first slit seal member **300** and would not significantly alter the operation of the seal assembly **10** as described below.

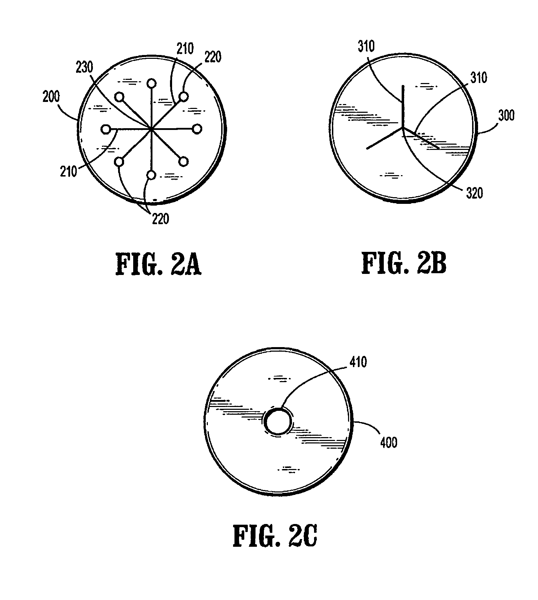

**Figs. 2A-C** are top plan views of the suture retaining member **200,** the slit seal member **300** and the instrument seal member **400,** respectively. Suture retaining member **200** includes a plurality of suture parks **220** configured to hold sutures **700** (see **Fig. 3**) and configured to help prevent the tangling of sutures **700.** The suture parks **220** are disposed adjacent to slits **210** defined in suture retaining member **200.** Slits **210** allow for the passage of sutures and/or surgical instrumentation therethrough. Slits **210** are configured such that at rest, slits **210** are in a closed position. Slits **210** may intersect or meet at intersection point **"K"** which may or may not be coincident with the longitudinal axis. Slits **210** communicate which may facilitate mounting and/or positioning of sutures relative to suture retainer member **210.** As shown in **Fig. 2A****,** the slits **210** of suture retaining member **200** extend radially from a center **230** of suture retaining member **200.** Each slit **210** extends into a suture park **220.** The suture parks **220** are configured such that a suture disposed therethrough will remain in that respective suture park **220.** The operation of suture retaining member **200** will be discussed in more detail below.

The embodiment of **Fig. 2A** shows eight slits **210** and eight corresponding suture parks **220.** However, it is envisioned and within the scope of the present disclosure that more or fewer slits **210** and/or suture parks **220** may be provided.

**Fig. 2B** is a top plan view of slit seal member **300.** Slit seal member **300** includes a plurality of slits **310** extending from a center **320** of slit seal member **300.** While three slits **310** are shown, it is envisioned and within the scope of the present disclosure that more or fewer slits **310** may be provided. Slits **310** allow for the passage of sutures and/or surgical instrumentation therethrough. In the absence of a surgical instrument disposed through seal assembly **10,** slit seal member **300** including slits **310** is in an at-rest or closed position. Slit seal member **300** is configured to create a substantially fluid-tight seal in the absence of an instrument passing therethrough. The operation of slit seal member **300** will be discussed in more detail below.

**Fig. 2C** is a top plan view of instrument seal member **400.** In the illustrated embodiments, instrument seal member **400** includes an aperture **410** configured to create a fluid-tight seal around an instrument inserted therethrough. The operation of instrument seal member **400** will be discussed in more detail below

Referring now to **Fig. 3****,** seal assembly **10** is shown in mechanical cooperation with cannula assembly **500.** A distal end **120** of housing **100** is shown monolithically formed with a cannula assembly 500. Seal assembly **10** may also be configured to mechanically engage cannula assembly **500** in a variety of ways including, but not limited to, through a bayonet lock or threaded connection. Cannula assembly **500** may include a second seal (not shown) which provides a substantially fluid-tight seal in the absence of a surgical instrument passing therethrough.

**Fig. 3** illustrates instrument **600** passing through seal assembly **10** and into cannula assembly **500.** Individual sutures **700** are disposed through seal assembly **10** and through suture parks **220.** Instrument **600** passes through slits **210** of suture retaining member **200,** slits **310** of slit seal member **300,** and aperture **410** of instrument seal member **400.** Instrument **600** extends into cannula assembly **500.**

The operation of seal assembly **10** will now be described in detail, with reference to **Figs. 1-3****.** Referring to **Fig. 1****,** seal assembly **10** is provided. Seal assembly **10** may be mounted onto cannula assembly **500 (****Fig. 3****).** Sutures **700** may be disposed through slits **210** of suture retaining member **200,** through slits **310** of slit seal member **300,** through aperture **410** of instrument seal member **400** and into cannula assembly **500.**

Once disposed as described above, a suture **700** may be pulled or dragged until disposed through a suture park **220.** Sutures **700** disposed through suture parks **220** are retained in suture parks **220,** thereby minimizing tangling of multiple sutures **700** disposed within housing **100** of seal assembly **10.** In addition, placement of sutures **700** within suture parks **220** will assist in identifying the respective sutures and their functioning. Positioning of sutures **700,** as described above, may be repeated until each suture **700** is disposed in a suture park **220.** Suture parks **220** are spaced apart and configured such that the likelihood of entanglement between a suture **700** disposed through a suture park **220** and another suture **700** disposed through another suture park **220** is reduced. Suture retaining member **200** may be fabricated from an elastomeric or resilient material.

An instrument **600** may be introduced into seal assembly **10** as follows. The instrument **600** is first inserted through slits **210** of suture retaining member **200.** Slits **210** expand from their at-rest position to accommodate instrument **600** therein. Slits **210** allow instrument **600** to pass therethrough, without displacing sutures **700** from suture parks **220.** As instrument **600** is farther advanced into access port **10,** instrument **600** passes through slits **310** of slit seal member **300.** Slits **310** expand to accept instrument **600.** As instrument **600** is advanced even farther, instrument **600** passes through aperture **410** of instrument seal member **400.** Aperture **410** creates a substantially fluid-tight seal around instrument **600** when disposed therethrough.

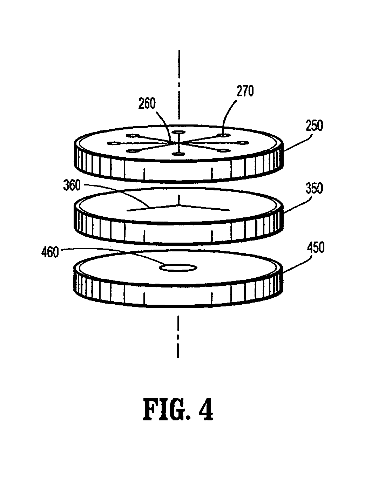

In the embodiment shown in **Fig. 4****,** an access port **20** of the present disclosure includes first portion **250,** second portion **350** and third portion **450.** Access port **20** is different from seal assembly **10** in that the three portions **250, 350** and **450** of access port **20** are integrally molded to form a seal assembly which is disposed within a housing. Seal assembly **10** includes a housing within which a suture retaining member, a slit seal member, and an instrument seal member are disposed, and not integrally formed therewith.

First portion **250** of access port 20 is shown positioned proximal to and adjacent second portion **350.** Second portion **350** is shown positioned proximal to and adjacent third portion **450.** First portion **250** includes a suture retaining member including slits **260** and suture parks **270.** Slits **260** and suture parks **270** of first portion **250** operate in substantially the same manner as slits **210** and suture parks **220** of suture retaining member **200,** as described above.

Second portion **350** includes a slit seal member including slits **360.** Slits **360** of second portion **350** operate in substantially the same manner as slits **310** of slit seal member **300,** as described above.

Third portion **450** includes an instrument seal member including an aperture **460.** Aperture **460** operates in substantially the same manner as instrument seal **410** of instrument seal member **400,** as described above.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

## Claims

1. A seal assembly configured for use with a cannula assembly, the seal assembly comprising:
a housing;
a suture retaining member disposed in mechanical cooperation with the housing, the suture retaining member configured to hold at least one suture and configured to help prevent the tangling of sutures;
a slit seal member disposed in mechanical cooperation with the housing, the slit seal member configured to create a substantially fluid-tight seal in the absence of a surgical instrument inserted therethrough; and
an instrument seal member disposed in mechanical cooperation with the housing, the instrument seal member configured to create a substantially fluid-tight seal around a surgical instrument inserted therethrough.

2. The seal assembly of claim 1, wherein the seal assembly comprises two slit seal members and wherein each slit seal member is disposed in mechanical cooperation with the housing.

3. The seal assembly of claim 1 or claim 2, wherein the suture retaining member is disposed in mechanical cooperation with a proximal portion of the housing.

4. The seal assembly of any preceding claim, wherein the slit seal member is disposed in mechanical cooperation with the housing adjacent and distal to the suture retaining member.

5. The seal assembly of any preceding claim, wherein the suture retaining member has a plurality of suture parks disposed therethrough.

6. The seal assembly of claim 5, wherein each suture park is disposed adjacent to a slit defined through the suture retaining member.

7. The seal assembly of any preceding claim, wherein a distal portion of the housing is configured to mechanically engage a cannula assembly.

8. The seal assembly of any preceding claim, wherein at least one of the suture retaining member, the slit seal member and the instrument seal member is fixedly attached to the housing.

9. The seal assembly of claim 8, wherein each of the suture retaining member, the slit seal member and the instrument seal member is fixedly attached to the housing.

10. An access port comprising:
a housing; and
a seal assembly disposed within the housing, the seal assembly including:
a first portion including a suture retaining member;
a second portion including a slit seal member; and
a third portion including an instrument seal member.

11. The access port of claim 10, wherein the housing is configured to mechanically engage a cannula assembly.

12. The access port of claim 10 or claim 11, wherein the suture retaining member has a plurality of suture parks configured to hold sutures and is configured to help prevent the tangling of sutures.

13. The access port of claim 12, wherein each suture park is disposed adjacent to a slit defined through the suture retaining member.
